# EUROPEAN PATENT SPECIFICATION

(11) **EP 2 520 264 B1**
(45) Date of publication and mention of the grant of the patent: **17.02.2016**
(21) Application number: 10840891.5
(22) Date of filing: 16.12.2010
(51) Int. Cl.: A61F 13/496, A61F 13/49, A61F 13/56

(54) **PANTS-TYPE WEARING ARTICLE**
HOSENFÖRMIGER TRAGEARTIKEL
ARTICLE À PORTER DE TYPE COUCHE-CULOTTE

(30) Priority: 28.12.2009 JP 2009298535
(43) Date of publication of application: 07.11.2012
(73) Proprietor: Unicharm Corporation, Ehime 799-0111 (JP)
(72) Inventor: MISHIMA, Yoshitaka, Kanonji-shi Kagawa 769-1602 (JP); KINOSHITA, Akiyoshi, Kanonji-shi Kagawa 769-1602 (JP)
(74) Representative: Murgatroyd, Susan Elizabeth
(86) International application number: PCT/JP2010/072713
(87) International publication number: WO 2011/081033

(56) References cited:
- EP-A1- 2 025 311
- EP-A1- 2 057 975
- WO-A1-2007/123445
- WO-A1-2009/019545
- WO-A1-2009/063293
- WO-A2-02/065961
- JP-A- 2003 528 695
- JP-A- 2004 121 389
- JP-A- 2004 121 389
- JP-A- 2004 298 401
- JP-A- 2006 175 007
- JP-A- 2009 535 088
- US-A- 4 670 012
- US-A1- 2001 034 512
- US-A1- 2003 078 558
- US-A1- 2008 009 816

## Description

### {Technical Field}

The present invention relates to pants-type wearing articles and particularly to pants-type wearing articles having front and rear waist regions connected with each other by the intermediary of separately prepared connecting members.

### {Background}

Pants-type wearing articles having front and rear waist regions connected with each other by the intermediary of connecting members prepared separately of these waist regions are known.

For example, the disposable diaper disclosed by JP 2004-121389 A (PTL 1) includes connecting sheets prepared separately of both a front waist region and a rear waist region to connect these two waist regions with each other. Each of the connecting sheets is formed with folds adapted to be expandable in a circumferential direction and these folds include the fastening means for temporarily holding the folds in a folded state. When it is desired to put the diaper on the wearer's body, keeping the folds in an expanded state, the wearer's legs may be guided through the diaper and then the folds may be fixed in the folded state again by using the fastening means. When it is desired to take the diaper off from the wearer's body, the folds may be kept in an expanded state to facilitate the diaper to be pulled down. In this known diaper, the connecting sheets have respective inner surfaces thereof bonded to the respective inner surfaces of the front and rear waist regions.

The pants-type wearing article disclosed by JP 2008-12115 A (PTL 2) includes connecting means adapted to connect front and rear waist regions along respective both lateral regions thereof in a separable and re-connectable fashion. The connecting means has a Z-shaped cross-section shape as viewed in a circumferential direction at one lateral region of the diaper and an inverted Z-shaped cross-section at the other lateral region. For example, in connecting means having the Z-shaped cross-section, a top segment of the Z-shape is permanently secured to an inner surface of one of the front and rear waist region, an intermediate segment and a bottom segment of the Z-shape are integrally bonded together, and an outer surface of the bottom segment is attached to an inner surface of the other of the front and rear waist regions in a separable and re-connectable fashion.

### (Citation List)

### (Patent Literature)

(PTL 1) JP 2004 - 121389 A
(PTL 2) JP 2008 - 12115 A

EP 2057975 A1 discloses a method for producing a closure element for disposable pants-like absorbent sanitary products formed by two parts basically symmetrical with respect to a central transverse plane, wherein each of the parts is able to form a side panel of an absorbent sanitary product. The method comprises the steps of: folding two continuous strips according to a configuration that comprises at least two parallel branches joined to one another by at least one fold; and fixing permanently respective end branches of the folded strips to a plane base layer, with said folded strips basically symmetrical respect to a central plane of the base layer.

EP 2025311 A1 discloses a closure element with elastic side panels for absorbent pants-like sanitary products. The element is formed starting from a strip at least partially made of elasticized material folded in transverse directions so as to form two parts, symmetrical relative to a central transverse plane, each of the parts being able to form a side panel of an absorbent sanitary product. The closure element comprises a first branch that extends continuously through the central transverse plane; two second branches connected to respective opposite ends of the first branch by means of respective first folds, distal relative to the central transverse plane, in which the first branch and the two second branches have respective mutually facing surfaces permanently fastened to each other; two third branches connected to respective ends of second branches by means of respective second folds, proximal and distanced relative to the central transverse plane; two fourth branches connected to respective ends of the two third branches in distal positions relative to said central transverse plane; and two fifth branches connected to respective ends of the fourth branches by means of respective third folds, proximal and distanced relative to the central transverse plane.

US 2008/009816 A1 discloses a pants-type wearing article including front and rear waist regions connected to each other by fastener means in a detachable and refastenable manner. Each of the fastener means comprises a sheet-like supporting element adapted to be folded in a Z-shape or an inverted Z-shape as the front and rear waist regions are put flat together and a fastener element. The supporting element has a top section, a bottom section and an intermediate section connecting the top and bottom sections. Between opposed surfaces of the bottom section and the intermediate section, a sheet-like liner section is provided so as to extend from an openable end of the bottom section along these opposed surfaces toward a joint end of the top section. The top section is bonded to the intermediate section by a first bonding zone while the liner section is bonded to the bottom section and the intermediate section by a second bonding zone. In the supporting element, the first bonding zone and the second bonding zone overlap each other in a height direction of the Z-shape with interposition of the intermediate section.

### (Summary)

### (Technical Problem)

In the disposable diaper disclosed in PTL 1, the inner surface of the connecting sheet is bonded to the inner surface of the front waist region and to the inner surface of the rear waist region. In such arrangement, the region in which the connecting sheet is bonded to the front waist region and the region in which the connecting sheet is bonded to the rear waist region protrude outward from the wearing article. These protrusions may unintentionally come in contact with the wearer's hands and cause the wearer to experience a cumbersome feeling. In addition, these protrusions form the diaper put on the wearer's body with queerly local bulges along the both sides of the diaper to deteriorate an appearance of the diaper.

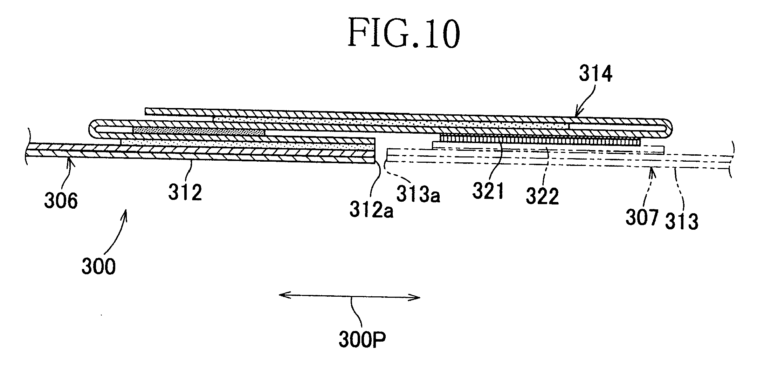

In the pants-type wearing article disclosed in PTL 2, the outer surfaces of the respective connecting means are bonded to the respective inner surfaces of the front and rear waist regions. With such arrangement, the regions in which the connecting means overlap the front and rear waist regions, respectively, should not protrude outward from the wearing article. However, the inventors of the present invention found that this known wearing article can not be free from problems as follows. Fig. 10 of the accompanying drawings exemplarily shows a sectional view taken along the waist regions' lateral zones of the pants-type wearing article disclosed in PTL 2. In this wearing article 300, a lateral zone 312 of a front waist region 306 is connected with a lateral zone 313 of a rear waist region 307 by the intermediary of a connecting means 314. While the connecting means 314 is permanently bonded to the lateral zone 312, the connecting means 314 is temporarily, i.e., in a separable and re-attachable manner, bonded to the lateral zone 313 by the intermediary of a fastener member. The fastener member includes hook elements 321 mounted on the connecting means 314 and loop elements 322 mounted on the lateral zone 313. In this way, an edge 312a of the lateral region 312 and an edge 313a of the lateral region 313 are relatively close to each other in a circumferential direction 300P and the hook elements 321 are mounted on the connecting means 314 in the vicinity of the edge 312a. Hence, when it is tried to put again the loop elements 322 having been disengaged from the hook elements 321 into engagement with the hook elements 321, undesirable situation possibly occurs such that the loop elements 322 should partially collide with the lateral region 312 and make it difficult for the loop elements 322 to come in close contact with the hook elements 321 and/or the area over which the loop elements 322 are put in engagement with the hook elements 321 should be insufficient for reliable connection. In this case, the loop elements 322 and the hook elements 321 which should have been reliably engaged together might be easily disengaged from each other. Even when the loop elements 322 and the hook elements 321 are reliably engaged together, if the lateral region 313 comes in contact with or overlaps the lateral region 312, the waist regions of the wearing article 300 may locally bulge in an awkward manner to deteriorate an appearance of the wearing article 300.

In view of the problem, it is an object of the present invention to provide a pants-type wearing article including connecting members adapted to connect front and rear waist regions along respective lateral regions thereof so that the front and rear waist regions may remain spaced from each other in a circumferential direction wherein the connecting members never cumber the wearer. Another object of the present invention is to provide a pants-type wearing article including the connecting members adapted to connect the front and rear waist regions so that the front and rear waist regions may remain spaced from each other in the circumferential direction wherein the connecting members may be separated from and re-connected to one of the front and rear waist regions and the lateral region of this waist region and the connecting members may be easily re-connected together.

### (Solution to Problem)

According to the present invention, there is provided a pants-type wearing article having a circumferential direction, a front-back direction and a vertical direction orthogonal to the front-back direction, the pants-type wearing article comprising a front waist region and a rear waist region opposed to each other in the front-back direction and respectively having lateral regions opposed to each other in the circumferential direction along which the front waist region and the rear waist region are connected together, wherein: the lateral region of the front waist region and the lateral region of the rear waist region are connected to each other by the intermediary of a sheet-like connecting member along at least one of both sides in the circumferential direction; the connecting member has its outer surface facing outward of the wearing article and is folded to form mountain folds convexly pointing outward of the wearing article and extending in the vertical direction and valley folds convexly pointing inward of the wearing article and extending in the vertical direction so that the mountain folds and the valley folds may alternate in the circumferential direction and may be expanded in the circumferential direction from the folded state as the connecting member is pulled in the circumferential direction; and the outer surface of the connecting member is attached to the inner surface of the front and rear waist regions; the connecting member is formed of sheet material which is folded in a zigzag manner with an edge pointing inwardly of the wearing article to define a plurality of layers, and a first layer of said layers is attached to the inner surface of one of the lateral regions; characterized in that:- said plurality of layers comprises the first layer through a fifth layer; the first layer is permanently bonded to the inner surface of the lateral region with a first adhesive and the fifth layer is permanently bonded to the lateral region with a second adhesive, and the fourth layer and the fifth layer are permanently bonded to each other with a third adhesive; and the first layer through the third layer of the sheet material are temporarily joined together in a first region and the first layer through the fourth layer of the sheet material are temporarily joined together in a second region.

According to one embodiment, the connecting member includes at least two mountain folds.

According to still another embodiment, the connecting member is elastically stretchable in the circumferential direction.

According to further another embodiment, the mountain folds of the connecting member in a folded state lie inner than respective edges of the lateral regions of the respective front and rear waist regions extending in the vertical direction.

According to an alternative embodiment, two or more types of the connecting member having different dimensions in the state expanded in the circumferential direction are used to connect the front and rear waist regions to obtain two or more types of the wearing article having different circumferential dimensions and thereby to obtain a group including these two more types of the wearing article.

### (Advantageous Effects of Invention)

In the pants-type wearing article according to the present invention, the lateral regions of the front waist region are connected with the lateral regions of the rear waist region by the intermediary of the sheet-like connecting members folded so as to be arranged alternately and expandable in the circumferential direction. With such arrangement, the dimension of the connecting member in the circumferential direction may be varied to vary the circumferential dimension of the wearing article. Furthermore, the outer surface of the connecting member facing outward of the wearing article is attached to the inner surface of the front and rear waist regions. With such unique arrangement, the connecting member as well as the front and rear waist regions provided with this connecting member should not protrude outward of the wearing article when the wearing article is put on the wearer's body.

### {Brief Description of Drawings}

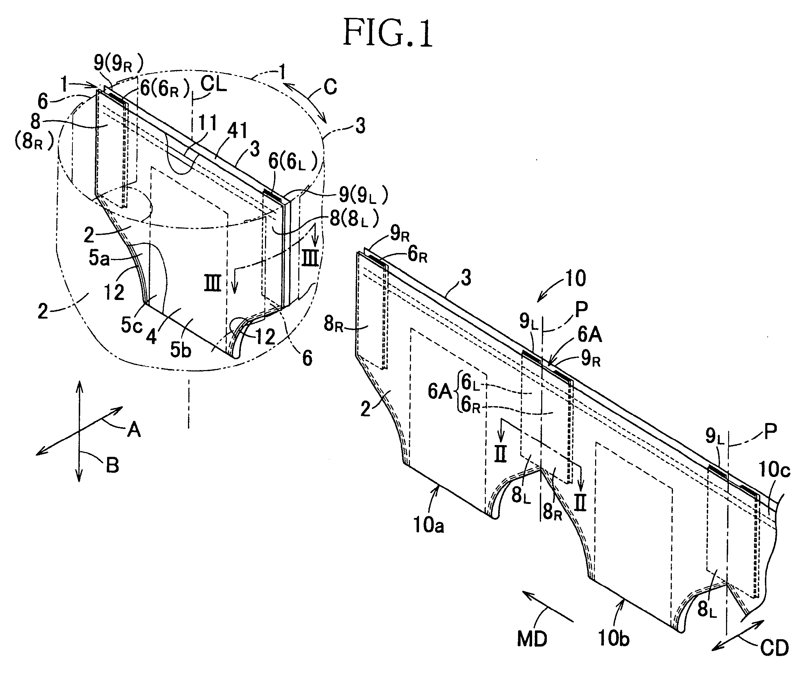

{Fig. 1}
   Fig. 1 is a perspective view showing a pants-type wearing article (pants-type diaper) and continuum thereof.
{Fig. 2}
   Fig. 2 is a sectional view taken along the line II-II in Fig. 1.
{Fig.3}
   Fig. 3 is a sectional view taken along the line **III-III** in Fig. 1, showing a connecting member in the course of being expanded.
{Fig. 4}
   Fig. 4 is a sectional view taken along the line **III-III** in Fig. 1, showing the connecting member having been completely expanded.
{Fig. 5}
   Fig. 5 is a view similar to Fig. 3 exemplarily showing another embodiment, which is outside the scope of the appended claims.
{Fig. 6}
   Fig. 6 is a view similar to Fig. 5 exemplarily showing another embodiment, which is outside the scope of the appended claims.
{Fig. 7}
   Fig. 7 is a view similar to Fig. 2 exemplarily showing yet another embodiment, which is outside the scope of the appended claims.
{Fig. 8}
   Fig. 8 is a schematic diagram exemplarily illustrating steps of making the connecting member according to one embodiment, which is outside the scope of the appended claims.
{Fig. 9}
   Fig. 9 is a schematic diagram exemplarily illustrating steps of making the connecting member according to another embodiment, which is outside the scope of the appended claims.
{Fig. 10}
   Fig. 10 is a diagram exemplarily illustrating the connecting member of prior art.

### {Description of Embodiments}

Details of the present invention will be more fully understood from the description of a pants-type diaper as one of the typical examples of the pants-type wearing article according to the present invention given hereunder with reference to the accompanying drawings.

Fig. 1 shows a pants-type diaper 1 and a part of a diaper continuum 10, i.e. diapers being contiguous one to another in the machine direction MD including intermediate products continuously made. The diaper 1 has a front waist region 2 and a rear waist region 3 opposed to each other in a front-back direction as described later in detail, and a crotch region 4 extended between and connected to these two waist regions 2, 3 in a vertical direction B as described later in detail wherein a connecting member 6 lies between the front waist region 2 and the rear waist region 3 in a circumferential direction C as described later in detail. Fig. 1 illustrates the diaper 1 having the front waist region 2 and the rear waist region 3 put flat together and the connecting member 6 tucked and collapsed between lateral region 8 of the front waist region 2 and lateral region 9 of the rear waist region 3. Both the front waist region 2 and the rear waist region 3 include waist elastic members 11 attached thereto under tension and in a contractible manner. The crotch region 4 includes leg elastic members 12 attached thereto under tension and in a contractible manner. In the crotch region 4, a bodily fluid-absorbent core 5c is sandwiched between a liquid-pervious topsheet 5a and a liquid-impervious backsheet 5b.

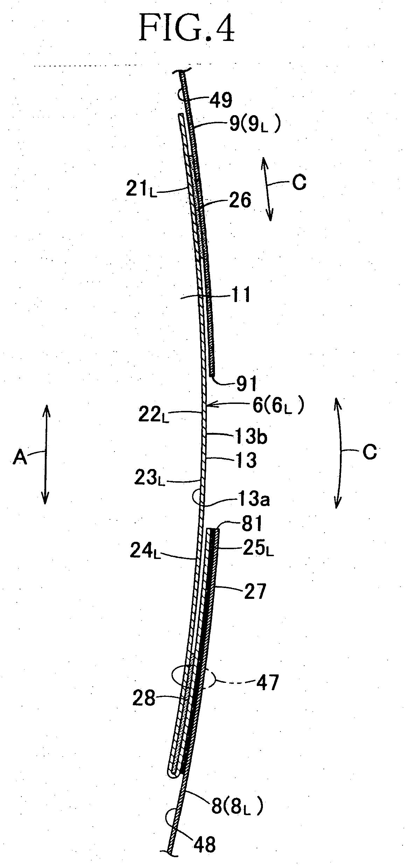

Referring to Fig. 1, chain double-dashed lines indicate the diaper 1 opened from the flattened state to be put on the wearer's body not illustrated. With the diaper 1 put on the wearer's body, both the front waist region 2 and the rear waist region 3 are curved to be put in close contact with the wearer's waist at a good fit (See Fig. 4 also). The lateral region 8 of the front waist region 2 and the lateral region 9 of the rear waist region 3 are spaced from each other and consequently the connecting member 6 having been tucked and collapsed between the lateral region 8 and the lateral region 9 is expanded in the circumferential direction C between these edges 8, 9. In Fig. 1, the front-back direction, the vertical direction and the circumferential direction are indicated by double-headed arrows A, B, C and, with respect to the diaper continuum 10, a direction in which the diaper continuum 10 runs and a cross direction orthogonal to the machine direction are indicated by an arrow MD and a double-headed arrow CD, respectively. The diaper 1 exemplarily shown is formed symmetrically about a center line CL bisecting a dimension in the machine direction MD. The lateral region 8 includes lateral regions 8_{L}, 8_{R} defined at both sides in the circumferential direction C, the lateral region 9 includes lateral regions 9_{L}, 9_{R} defined on both sides in the circumferential direction C, and the connecting member 6 including a connecting member 6_{L}, 6_{R}. Letters _{L} and _{R} suffixed to the reference numerals means left side and right side for the wearer. It should be understood that the lateral region 9 suffixed with neither _{L} nor _{R} means the lateral region not discriminated between left side and right side.

The diaper continuum 10 shown in Fig. 1 includes the intermediate products 10a, 10b, ... continuously arranged in the machine direction MD so as to become the individual diapers 1 successively wherein the lateral region 8_{L} and the lateral region 9_{L} of the intermediate product 10a are contiguous to the lateral region 8_{R} and the lateral region 9_{R} of the intermediate product 10b. In the machine direction MD, the connecting member 6_{L} of the intermediate product 10a is contiguous to the connecting member 6_{R} of the intermediate product 10b and these two connecting members form a composite connecting member 6A.

The intermediate product 10a and the intermediate product 10b are cut off along an imaginary line P and, in consequence, the lateral region 8_{L} is separated from the lateral region 8_{R}, the lateral region 9_{L} is separated from the lateral region 9_{R}, and the composite connecting member 6A is separated into the connecting member 6_{L} and the connecting member 6_{R}. In this way, an individual diaper 1 is obtained from the intermediate product 10a. It should be appreciated that the imaginary line P will be sometimes designated as the cutting line P in the later description.

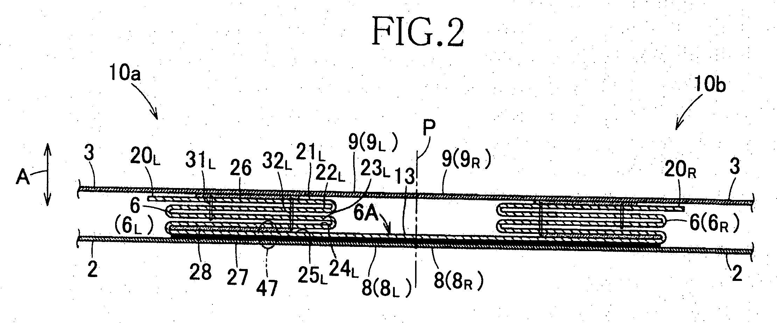

Fig. 2 is a sectional view taken along the line II-II in Fig. 1. Referring to Fig. 2, the lateral regions 8_{L}, 8_{R}, 9_{L}, 9_{R} of the respective intermediate products 10a, 10b are formed of a sheet material, for example, made of a nonwoven fabric, a woven fabric, a plastic film, a laminate of two or more kinds of nonwoven fabrics or a laminate of nonwoven fabrics or woven fabrics and plastic films. Referring to Fig. 1, for example, a nonwoven fabric defining the topsheet 5a and a plastic film defining the backsheet 5b may be permanently bonded to each other outside a peripheral edge of the core 5c to define the lateral regions 8_{L}, 8_{R}, 9_{L}, 9_{R}. The composite connecting member 6A also may be formed of sheet material 13, for example, made of a nonwoven fabric, a woven fabric, a plastic film, a laminate of two or more kinds of nonwoven fabrics or a laminate of nonwoven fabrics or a woven fabric and a plastic film wherein the nonwoven fabric and/or the woven fabric preferably contains a thermoplastic synthetic resin. The composite connecting member 6A is symmetric about the imaginary line P and therefore a cross section structure of the composite connecting member 6A may be understood on the basis of a cross section structure of the half thereof occupying the intermediate product 10a, i.e., the connecting member 6_{L}. The connecting member 6_{L} has the following construction. In the intermediate product 10a, the sheet material 13 is folded in a zigzag manner with an edge 20_{L} pointing inwardly of the diaper 1 to define first through fifth layers 21_{L} - 25_{L}. The first layer 21_{L} is permanently bonded to the inner surface of the lateral region 9_{L} with a first adhesive 26. The fifth layer 25_{L} is permanently bonded to the lateral region 8_{L} with a second adhesive 27. The fourth layer 24_{L} and the fifth layer 25_{L} are permanently bonded to each other with a third adhesive 28. The first layer 21_{L} through the third layer 23_{L} of the sheet material 13 are temporarily joined together in a first region 31_{L} and the first layer 21_{L} through the fourth layer 24_{L} of the sheet material 13 are temporarily joined together in a second region 32_{L}. The first and second regions 31_{L}, 32_{L} function as temporary fastener means adapted to keep the connecting member 6_{L} in a folded state until the diaper 1 is put on the wearer's body. To temporarily join the first and second regions 31_{L}, 32_{L} of the sheet material 13 to each other in the first and second regions 31_{L}, 32_{L}, hooked pins (not shown) may be stuck into the first through three layers 21_{L} - 23_{L} or the first through fourth layers 21_{L} - 24_{L} and fibers or films forming the respective layers may be mechanically intertangled or the heated pins may be stuck into the respective layers and thereby the fibers or films may be fusion bonded together.

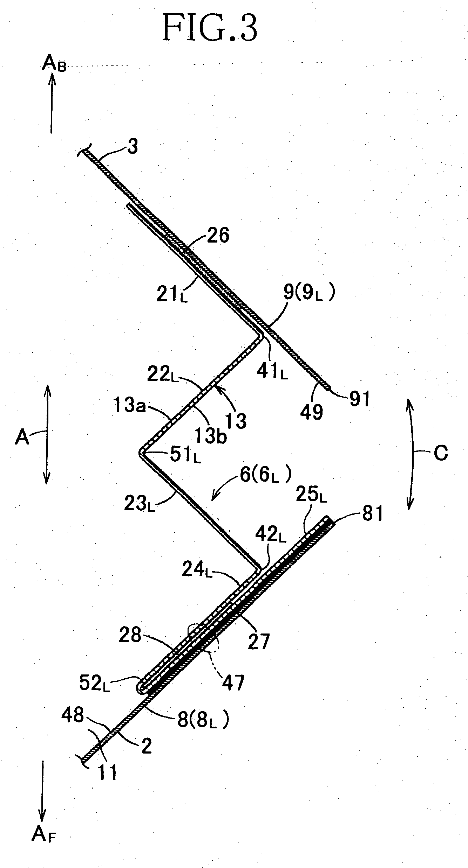

Figs. 3 and 4 are sectional views taken along the line III-III in Fig. 1, illustrating a course of moving the front and rear waist regions 2, 3 having put flat together in the front-back direction A and thereby broadening the waist-opening to put the diaper 1 including the connecting member 6_{L}. It should be understood here that Fig. 3 shows a state in which the waist-opening begins to be broadened as the connecting member 6_{L} is expanded and Fig. 4 shows a state in which the connecting member 6_{L} is fully expanded and thereby the waist-opening 11 has been completely broadened. Specifically, putting the wearer's or caregiver's hands between the front waist region 2 and the rear waist region 3, the front waist region 2 may be moved forward as indicated by an arrow A_{F} and the rear waist region 3 may be moved backward as indicated by an arrow A_{B} to broaden the waist-opening 11 which has been closed in Fig. 1. Thereupon the connecting member 6_{L} is pulled by the lateral region 8_{L} and the lateral region 9_{L} in the front-back direction A until the temporary fastening effect in the first region 31_{L} and the second region 32_{L} are released and thereby the connecting member 6_{L} is expanded in the front-back direction A. Referring to Fig. 3 showing the connecting member 6 in the course of being expanded and Fig. 2, the connecting member 6_{L} has a first mountain fold 41_{L} defined between the first layer 21_{L} and the second layer 22_{L} and convexly pointing outward of the diaper 1, a first valley fold 51_{L} defined between the second layer 22_{L} and the third layer 23_{L} and convexly pointing inward of the diaper 1 and a second mountain fold 42_{L} defined between the third layer 23_{L} and the fourth layer 24_{L} and pointing outward of the diaper 1. A second valley fold 52_{L} defined between the fourth layer 24_{L} and the fifth layer 25_{L} is kept closed under the effect of the third adhesive 28. While not shown, also between the lateral region 8_{R} and the lateral region 9_{R} of the diaper 1, the connecting member 6_{R} is expanded in the front-back direction A as the effect of the associated temporary fastening is released. In this way, the front and rear waist regions 2, 3 cooperate with the connecting members 6_{L}, 6_{R} to define substantially the circular waist-opening 11 as an indicated by an imaginary line in Fig. 1. The first and second mountain folds 41_{L}, 42_{L} are located on the inside of an edge 81 of the lateral region 8_{L} extending in the longitudinal direction B and an edge 91 of the lateral region 9_{L} extending in the longitudinal direction B, respectively. With such arrangement, a width dimension of the diaper 1 flattened as shown in Fig. 1 should not be enlarged by the presence of the connecting member 6_{L}.

Referring to Fig. 4, the lateral region 8_{L} and the lateral region 9_{L} are connected by the intermediary of the expanded connecting member 6_{L} in a similar fashion to the diaper 1 indicated by imaginary lines in Fig. 1. In the first layer 21_{L} of the connecting member 6_{L} for the most part, an outer surface 13b of the sheet material 13 defining the outer surface of the first layer 21_{L} is bonded to an inner surface 49 of the lateral region 9_{L} with the first adhesive 26. The second layer 22_{L} and the third layer 23_{L} lie between the lateral region 9_{L} and the lateral region 8_{L}. In the fourth layer 24_{L}, the outer surface 13b of the sheet material 13 is bonded to the outer surface 13b of the sheet material 13 in the fifth layer 25_{L} with the third adhesive 28. In the fifth layer 25_{L}, its surface contiguous to the inner surface 13a of the sheet material 13 is bonded to an inner surface 48 of the lateral region 8_{L} with the second adhesive 27. The second adhesive 27 and the third adhesive 28 are coated to the sheet material in a manner that these two adhesive layers may overlap each other by the intermediary of the fifth layer 25_{L}. More specifically, the second adhesive 27 and the third adhesive 28 are illustrated in Fig. 4 so as to overlap each other in a direction intersecting with the front-back direction A and are illustrated in Fig. 2 so as to overlap each other in the front-back direction A. According to such embodiment characterized in that the lateral regions 8_{L}, 9_{L} are bonded to the connecting member 6_{L}, the dimension or dimensions of the second layer 22_{L} and/or the third layer 23_{L} in the front-back direction A as viewed in Fig. 4 may be selectively varied to vary a distance between the lateral region 8_{L} and the lateral region 9_{L} in the front-back direction A, in other words, the distance therebetween in the circumferential direction C. In this way, the dimension of the diaper 1 in the circumferential direction C can be selectively varied merely by varying the dimension of the connecting member 6_{L} in the front-back direction A as viewed in Fig. 4 and it is unnecessary for this purpose to vary respective shapes and sizes of the front waist region 2 and the rear waist region 3 in the course of making the diaper 1. This is true for the connecting member 6_{R} (See Fig. 2) . The same effect can be obtained also without departing from the scope of the invention by dimensionally fixing one of the connecting members 6_{L}, 6_{R} and by dimensionally varying the other of the connecting members 6_{L}, 6_{R}. It is also possible without departing from the scope of the present invention to prepare differentially dimensioned two or more connecting members 6_{L} and/or connecting members 6_{R} and to use these connecting members 6_{L} and/or connecting members 6_{R} selectively with the uniformly dimensioned front waist regions 2 and/or the uniformly dimensioned rear waist regions 3 to obtain a group of the diapers respectively having the different dimensions in the circumferential direction C.

Referring also to Fig. 4, not a peel force but a shearing force is generated between the lateral region 8_{L} and the connecting member 6_{L} as well as between the lateral region 9_{L} and the connecting member 6_{R} as the lateral regions 8_{L}, 9_{R} are pulled in the front-back direction A or the circumferential direction C. Consequentially, in the course of putting the diaper 1 on the wearer's body or during use of the diaper 1, the connecting member 6_{L} may not be readily peeled off from the lateral regions 8_{L}, 9_{L}.

Referring also to Fig. 4, in the fourth layer 24_{L} and the fifth layer 25_{L} of the connecting member 6_{L}, the outer surface 13b of the sheet material 13 is bonded to the inner surface 48 of the lateral region 8_{L} with the second adhesive 27 and the third adhesive 28. With such arrangement, the expression that the inner surface 13a of the sheet material 13 in the fifth layer 25_{L} is bonded to the inner surface 48 of the lateral region 8_{L} practically means that the sheet material 13 of the connecting member 6_{L} has its outer surface 13b bonded to the inner surface 48 of the lateral region 8_{L}. With the diaper 1 put on the wearer' s body, all these connecting member 6_{L}, lateral region 8_{L} and lateral region 9_{L} joined one to another in this manner extend in the circumferential direction C and any one of them should not protrude outward in a radial direction of the waist-opening 11. More specifically, the diaper 1 is free from the problem possibly occurring, for example, due to the arrangement in which the inner surface 13a of the connecting member 6_{L} and the inner surface 49 of the lateral region 9_{L} are put flat and bonded together, i.e., the problem that the connecting member 6_{L} and the lateral region 9_{L} might protrude outward in the radial direction of the waist-opening 11 and irritate the diaper wearer's skin. As the sheet material 13, the sheet material being elastically stretchable in the circumferential direction C may be used. The connecting members 6_{L}, 6_{R} made of such sheet material 13 may be stretched in the circumferential direction C after having been expanded from the folded state to adapt the diaper 1 to various wearers having different waist sizes, respectively.

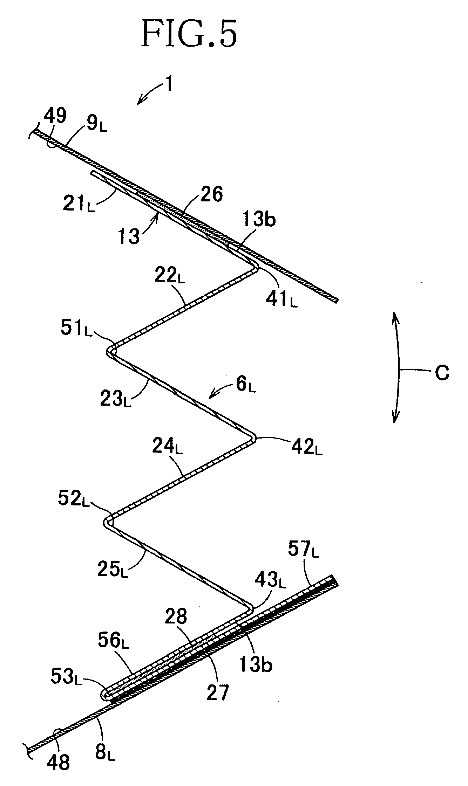

Fig. 5 is a view similar to Fig. 3, exemplarily showing another embodiment. While the diameter 1 shown in Fig. 5 is shaped symmetrically about the center line CL (See Fig. 1) as in the diaper 1 in Fig. 1, the diaper 1 according to this embodiment includes the connecting member 6_{L} having an arrangement different from that of the connecting member 6_{L} exemplarily shown in Fig. 3. The connecting member 6_{L} shown in Fig. 5 is folded to form, in addition to the first layer 21_{L}, the second layer 22_{L}, the third layer 23_{L}, the fourth layer 24_{L} and the fifth layer 25_{L}, a sixth layer 56_{L} and a seventh layer 57_{L} so that the connecting member 6_{L} includes, in addition to the first mountain fold 41_{L}, the second mountain fold 42_{L}, a third mountain fold 43_{L} and, in addition to the first valley fold 51_{L} and the second valley fold 52_{L}, a third valley fold 53_{L}. In the first layer 21_{L}, the outer surface 13b of the sheet material 13 is bonded to the inner surface 49 of the lateral region 9_{L} with the first adhesive 26 and the outer surface 13b of the sheet material 13 in the sixth layer 56_{L} is bonded to the inner surface 48 of the lateral region 8_{L} by the intermediary of the third adhesive, a seventh layer 57_{L} and the second adhesive 27. The connecting member 6_{L} shown in Fig. 5 is suitable for the case in which it is desired to enlarge a distance between the lateral region 8_{L} and the lateral region 9_{L}. Even if the connecting member 6_{L} has its total length in the circumferential direction not so long, a rectilinear distance from the apex of the mountain fold to the bottom of the valley fold can be reduced by folding the connecting member 6_{L} so that three or more mountain folds may be formed.

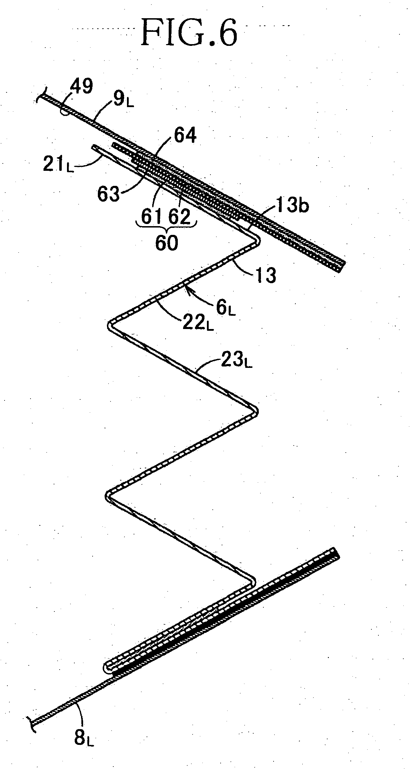

Fig. 6 is a view similar to Fig. 5, exemplarily showing still another embodiment. While the connecting member 6_{L} shown in Fig. 6 is substantially similar to the connecting member 6_{L} so long as the sectional shape is concerned, the first layer 21_{L} is attached to the lateral region 9_{L} by the intermediary of a fastener 62 adapted to function in a repetitively separable and re-attachable fashion. A typical example of the fastener 60 is so-called a mechanical fastener including a hook member 61 and a loop member 62. Referring to Fig. 6, the hook member 61 is attached to the outer surface 13b of the sheet material 13 forming the first layer 21_{L} by the intermediary of an adhesive 63. The loop member 62 serving as a counterpart for the hook member 61 is bonded to the inner surface 49 of the lateral region 9_{L} with adhesive 64. The connecting member 6_{L} and the lateral region 9_{L} can be repeatedly opened (or expanded) and collapsed as the hook member 61 and the loop member 62 are repeatedly disengaged from and engaged with each other. In the similar fashion to the case exemplarily shown in Figs. 3, 4 and 5, the lateral region 9_{L} and the lateral region 8_{L} are adequately spaced from each other by the intermediary of the second layer 22_{L} and the third layer 23_{L} of the connecting member 6_{L}. With such arrangement, regardless of the site in the loop member 62 selected to be put into engagement with the hook member 61, the lateral region 9_{L} and the lateral region 8_{L} should not come in contact with each other or overlap each other, making it difficult to put the hook member 61 quickly into engagement with the loop member 62. While the exemplarily illustrated connecting member 6_{L} has the first layer 21_{L} and the lateral region 9_{L} adapted to be repeatedly joined to and separated from each other, it is possible to implement the present invention in a manner that the first layer 21_{L} is adapted to be repeatedly joined to and separated from the lateral region 9ₗ. It is also possible to implement the present invention by using the connecting member 6_{R} which is symmetric to the connecting member 6_{L} shown in Fig. 6. Furthermore, it is possible to implement the present invention wherein only a combination of the connecting member 6_{R} and the lateral region 9_{R} can be repeatedly joined to and separated from each other.

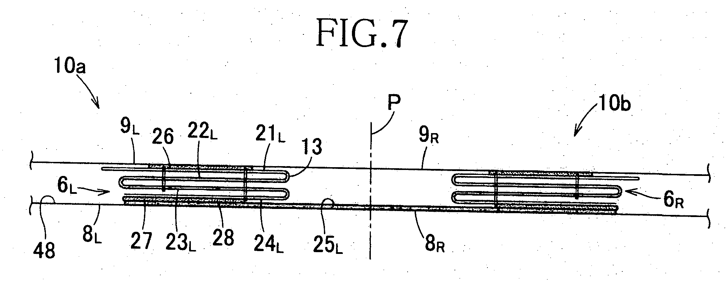

Fig. 7 is a view similar to Fig. 2, exemplarily showing yet another embodiment. The first layer 21_{L} - the fourth layer 24_{L} lying between the lateral region 8_{L} and the lateral region 9_{L} of the intermediate 10a of the diaper 1 shown in Fig. 7 are respectively formed of a peel-resistant two-ply sheet material 13 and only the fifth layer 25_{L} is formed of a single layer of the sheet material 13. The two-ply sheet material 13 may be made peel-resistant by bonding two layers of the individual sheet material 13 using an adhesive such as a hot melt adhesive or a heat-embossing treatment so as to fusion-bond thermoplastic synthetic fibers or a plastic film contained in the sheet material 13. The connecting member 6_{L} constructed as illustrated in Fig. 7 is preferably employed when a basis mass of the sheet material 13 is insufficient to obtain the strength required for the connecting member 6_{L} with a single layer of the sheet material 13.

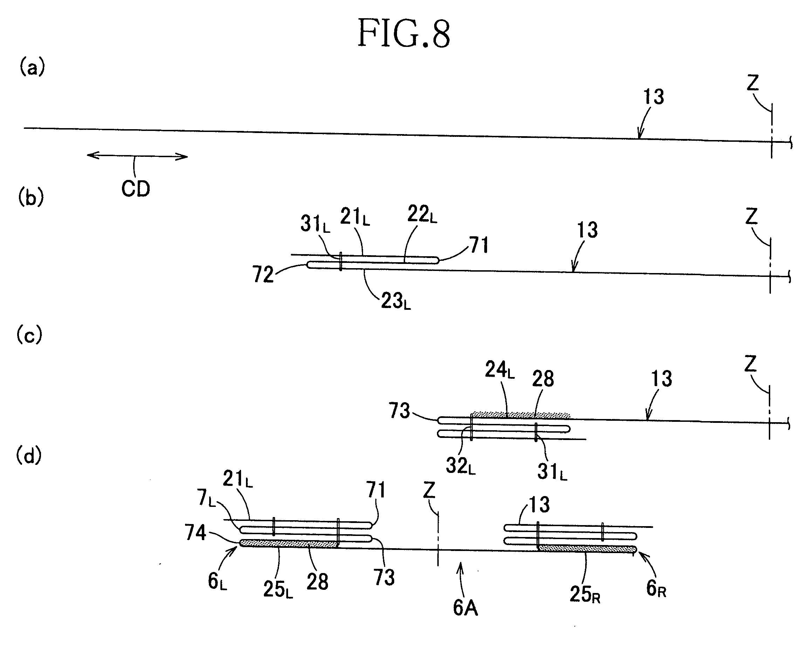

Fig. 8 is a schematic diagram illustrating steps (a) - (d) to obtain the composite connecting member 6A exemplarily shown in Fig. 2 from the sheet material 3. In the step (a), the continuous sheet material 13 for the composite connecting member 6A runs in the machine direction (not shown) and, in Fig. 8, the continuous sheet material 13 is illustrated in its sectional view taken in the cross direction CD. The cross direction CD is the direction orthogonal to the machine direction and corresponds to the width direction of the sheet material 13. A center line Z is the line bisecting the width dimension of the sheet material 13.

In the step (b), the both lateral regions of the sheet material 13 are folded along first and second fold lines 71, 72, respectively, so that a Z-shape and an inverted Z-shape may be defined symmetrically about the center line Z and thereby the first layer 21_{L}, 21_{R}, the second layer 22_{L}, 22_{R}, and the third layer 23_{L}, 23_{R} may be formed. The first layer 21_{L} - the third layer 23_{L} and the first layer 21_{R} - the third layer 23_{R} are respectively stuck with heated pins (not shown) to form the first region 31_{L}, 31_{R} serving for temporary fastening. With respect to the step (b), it should be appreciated that the inverted Z-shape and the first layer 21_{R} - the third layer 23_{R} are not illustrated in Fig. 8.

In the step (c), the sheet material 13 is folded along a third fold line 73 to form the fourth layer 24_{L}, 24_{R}. Then, the first layer 21_{L} - the fourth layer 24_{L} and the first layer 21_{R} - the fourth layer 24_{R} are respectively stuck with heated pins (not shown) to form the second region 32_{L}, 32_{R} serving for temporary fastening. The fourth layer 24_{L}, 24_{R} are coated with the third adhesive 28. With respect to the step (c), the first layer 21_{R} - the fourth layer 24_{R}, the first and second regions 31_{R}, 32_{R} are not illustrated in Fig. 8.

In the step (d), the sheet material 13 is folded along a fourth fold line 74 to form the fifth layer 25_{L}, 25_{R}, and the fourth layer 24_{L}, 24_{R} are bonded to the fifth layer 25_{L}, 25_{R}, respectively, with the third adhesive 28.

The sheet material 13 having been folded in the steps (a) - (d) is continuous in the machine direction and may be cut into a desired dimension in the machine direction to obtain the composite connecting members 6A as shown in Fig. 2. These composite connecting members 6A are bonded to the respective inner surfaces of the lateral regions 8_{L}, 8_{R}, 9_{L}, 9_{R} of the intermediates 10a, 10b,... in the continuum 10 of the diaper 10 with the first adhesive 26 and the second adhesive 27.

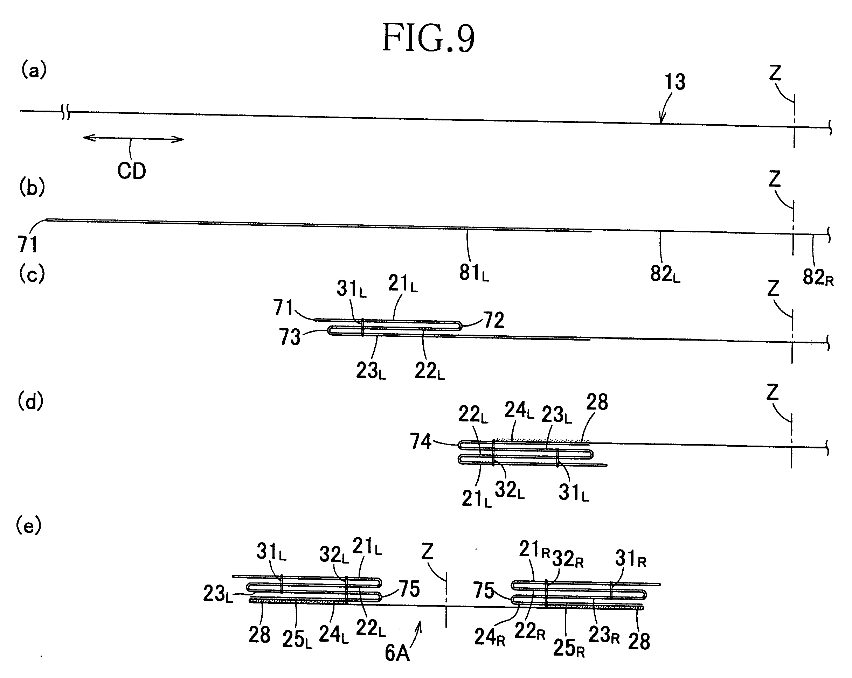

Fig. 9 is a schematic diagram illustrating steps (a) - (e) to obtain the composite connecting member 6A exemplarily shown in Fig. 8 from the sheet material 13. In the step (a), the continuous sheet material 13 for the composite connecting member 6A runs in the machine direction (not shown) and, in Fig. 8, the continuous sheet material 13 is illustrated in its sectional view taken in the cross direction CD.

In the step (b), the both lateral regions of the sheet material 13 are folded along the first fold line 71 to form lateral regions 81_{L}, 81_{R} defined by a two-ply sheet material 13 and intermediate regions 82_{L}, 82_{R} define by the single layer of the sheet material 13. The lateral regions 81_{L}, 81_{R} are heat-embossed so that the two-ply sheet material 13 may become peel-resistant. With respect to this step (b), the lateral region 81_{R} is not illustrated in Fig. 9.

In the step (c), the sheet material 13 is folded along second and third fold lines 72, 73 so as to define a Z-shape and an inverted Z-shape on both sides of the center line Z and thereby to form the first layer 21_{L}, 21_{R}, the second layer 22_{L}, 22_{R}, and the third layer 23_{L}, 23_{R}. The first layer 21_{L} - the first layer 21_{R} - the third layer 23_{R} are respectively formed with the first region 31_{L}, 31_{R} serving for temporary fastening. With respect to this step (b), the lateral region 81_{R} is not illustrated in Fig. 9.

In the step (d), the sheet material 13 is folded along a fourth fold line 74 to form the fourth layer 24_{L}, 24_{R}. The first layer 21_{L} - the fourth layer 24_{L} and the first layer 21_{R} - the fourth layer 24_{R} are respectively formed with the second region 32_{L}. 32_{R} serving for temporary fastening. The fourth layer 24_{L}, 24_{R} is coated with the third adhesive 28. With respect to this step (d), the first layer 21_{R} - the fourth layer 24_{R} are not illustrated in Fig. 9.

In the step (e), the sheet material 13 is folded along a fifth fold line 75 to form the fifth layer 25_{L}, 25_{R}, and the fourth layer 24_{L} and the fifth layer 25_{L} are bonded together by means of the third adhesive 28 to obtain the composite connecting member 6A having a sectional construction as exemplarily shown in Fig 7. While the composite connecting member 6A is illustrated in Fig. 9 to be continuous in the machine direction, such continuous composite connecting member 6a is cut into a desired dimension to obtain the individual composite connecting member 6A. The composite connecting member 6A may be used in the same manner as the composite connecting member 6A shown in Fig. 8.

The present invention having been described hereinabove on the basis of the pants-type diaper 1 as one of the typical examples thereof is applicable also to the other pants-type wearing article such as toilet-training pants, pants for incontinent patient or sanitary shorts.

Based on the description having been made above, essential features of the diaper 1 as the typical example of the pants-type wearing article according to the present invention may be summarized as follows:
1. The wearing article having been described hereinabove as the typical example is the pants-type diaper 1 having the circumferential direction C, the front-back direction A and the vertical direction B orthogonal to the front-back direction A and including the front waist region 2 and the rear waist region 3 opposed to each other in the front-back direction A and respectively having lateral regions 8_{L}, 8_{R} opposed in the circumferential direction C and lateral regions 9_{L}, 9_{R} opposed in the circumferential direction C along which these front and rear waist regions 2, 3 are joined together.
2. This diaper 1 is characterized in that: Along at least one of the lateral regions opposed to each other in the circumferential direction C, the lateral region 8_{L}, 8_{R} of the front waist region 2 and the lateral region 9_{L}, 9_{R} of the rear waist region 3 are connected to each other by the intermediary of the sheet-like connecting member 6_{L}, 6_{R}. The connecting member 6_{L}, 6_{R} has the outer surface 13b facing outward of the diaper 1 and folded in the circumferential direction C in expandable fashion to form the mountain folds 41, 42 and the valley folds 51, 52. The mountain folds 41, 42 convexly point outward of the diaper 1 and extend in the vertical direction and the valley folds 51, 52 convexly point inward of the diaper 1 and extend in the vertical direction wherein these mountain folds and valley folds alternate in the circumferential direction. The connecting member 6_{L}, 6_{R} is expanded in the circumferential direction C as the connecting member 6_{L}, 6_{R} is pulled in the circumferential direction C. The outer surface 13b of the connecting member 6_{L}, 6_{R} is attached to the inner surface 48, 49 of the diaper 1.
3. The connecting member 6_{L}, 6_{R} may include at least two mountain folds 41, 42, In the diaper 1 having such connecting member 6_{L}, 6_{R}, the expandable valley fold may be formed between the mountain folds 41, 42.
4. The connecting member 6_{L}, 6_{R} may be attached to at least one of the front and rear waist regions 2, 3 in a separable and re-connectable fashion. In the diaper 1 having such connecting member 6_{L}, 6_{R}, it is possible to disconnect the front and rear waist regions 2, 3 from each other when it is desired to put the diaper 1 on the wearer's body or to on pull off the diaper 1 from the wearer's body.
5. The connecting member 6_{L}, 6_{R} may be made to be elastically stretchable in the circumferential direction C. The diaper 1 having such connecting member 6_{L}, 6_{R} can adapt to wearers having a wide range of waist dimension.
6. The connecting member 6_{L}, 6_{R} in a folded state may be temporarily fastened so as to be temporarily maintained in such folded state. In the diaper 1 having such connecting member 6_{L}, 6_{R}, the connecting member should not unintentionally be expanded in the course of making the connecting member or delivering the diaper.
7. Apices of the mountain folds 41, 42 of the connecting member 6_{L}, 6_{R} in a folded state may lie inside the edges of 81, 91 of the respective lateral regions 8_{L}, 8_{R}, 9_{L}, 9_{R} extending in the vertical direction B. In such diaper 1, the width dimension of the flattened diaper should not be added with the dimension of the lateral regions and therefore the width dimension of the flattened diaper correspondingly reduced and the dimension of the diaper's package also can be reduced.
8. By using two or more types of the connecting member 6_{L}, 6_{R} having different dimensions in the state expanded in the circumferential direction C to connect the front and rear waist regions 2, 3, it is possible to make a group of two or more types. These two or more types of the connecting member 6_{L}, 6_{R} may be selectively used to facilitate to a group of diapers 1 having different waist dimensions to be obtained.

### {Reference Signs List}

- 1: wearing article (diaper)
- 2: front waist region
- 3: rear waist region
- 6, 6_{L}, 6_{R}: connecting member
- 8, 8_{L}, 8_{R}: lateral regions
- 9, 9_{L}, 9_{R}: lateral regions
- 13b: outer surface
- 41_{L}: mountain fold
- 42_{L}: mountain fold
- 43_{L}: mountain fold
- 48: inner surface
- 49: inner surface
- 51_{L}: valley fold
- 52_{L}: valley fold
- 53_{L}: valley fold
- 81: edge
- 91: edge
- A: front-back direction
- B: vertical direction
- C: circumferential direction

## Claims

1. A pants-type wearing article (1) having a circumferential direction (C), a front-back direction (A) and a vertical direction (B) orthogonal to the front-back direction (A), the pants-type wearing article (1) comprising a front waist region (2) and a rear waist region (3) opposed to each other in the front-back direction (A) and respectively having lateral regions (8, 9) opposed to each other in the circumferential direction (C) along which the front waist region (2) and the rear waist region (3) are connected together, wherein:
the lateral region (8) of the front waist region (2) and the lateral region (9) of the rear waist region (3) are connected to each other by the intermediary of a sheet-like connecting member (6) along at least one of both sides in the circumferential direction (C);
the connecting member(6) has its outer surface (13b) facing outward of the wearing article (1) and is folded to form mountain folds (41, 42, 43) convexly pointing outward of the wearing article (1) and extending in the vertical direction (B) and valley folds (51, 52, 53) convexly pointing inward of the wearing article (1) and extending in the vertical direction (B) so that the mountain folds (41, 42, 43) and the valley folds (51, 52, 53) may alternate in the circumferential direction (C) and may be expanded in the circumferential direction(C) from the folded state as the connecting member(6) is pulled in the circumferential direction(C); and
the outer surface. (13b) of the connecting member (6) is attached to the inner surface (48, 49) of the front and rear waist regions (2, 3);
the connecting member (6) is formed of sheet material (13) which is folded in a zigzag manner with an edge (20) pointing inwardly of the wearing article (1) to define a plurality of layers (21-25), and a first layer (21) of said layers (21-25) is attached to the inner surface of one (9) of the lateral regions;
**characterized in that**:-
said plurality of layers (21-25) comprise the first layer (21) through a fifth layer (25);
the first layer (21) is permanently bonded to the inner surface of the lateral region (9) with a first adhesive (26) and the fifth layer (25) is permanently bonded to the lateral region (8) with a second adhesive (27), and the fourth layer (24) and the fifth layer (25) are permanently bonded to each other with a third adhesive (28); and
the first layer (21) through the third layer (23) of the sheet material (13) are temporarily joined together in a first region (31) and the first layer (21) through the fourth layer (24) of the sheet material (13) are temporarily joined together in a second region (32).

2. The wearing article defined by Claim 1, wherein the connecting member (6) includes at least two mountain folds (41, 42, 43).

3. The wearing article defined by Claim 1 or 2, wherein the connecting member (6) is elastically stretchable in the circumferential direction (C).

4. The wearing article(1) defined by any one of Claims 1 through 3, wherein the mountain folds (41,42,43) of the connecting member (6) in a folded state lie inner than respective edges of the lateral regions (8, 9) of the respective front and rear waist regions (2, 3) extending in the vertical direction(B).

5. The wearing article defined by any one of Claims 1 through 4, wherein two or more types of the connecting member (6) having different dimensions in the state expanded in the circumferential direction(C) are used to connect the front and rear waist regions (2, 3) to obtain two or more types of the wearing article (1) having different circumferential dimensions and thereby to obtain a group including the two or more types of the wearing article (1).

## Patentansprüche

1. Hosenartiger Trageartikel (1), der eine Umfangsrichtung (C), eine Vor-Rück-Richtung (A) und eine senkrechte Richtung (B), die senkrecht zur Vor-Rück-Richtung (A) ist, hat, wobei der hosenartige Trageartikel (1) einen vorderen Hüftbereich (2) und einen hinteren Hüftbereich (3) hat, die sich in der Vor-Rück-Richtung (A) gegenüberliegen und jeweils seitliche Bereiche (8, 9) aufweisen, die sich in der Umfangsrichtung (C) gegenüberliegen, entlang derer der vordere Hüftbereich (2) und der hintere Hüftbereich (3) miteinander verbunden werden, wobei:
der seitliche Bereich (8) des vorderen Hüftbereichs (2) und der seitliche Bereich (9) des hinteren Hüftbereichs (3) unter Vermittlung eines bahnartiges Verbindungselements (6) entlang mindestens einer der beiden Seiten in der Umfangsrichtung (C) miteinander verbunden sind;
von dem Verbindungselement (6) die Außenoberfläche (13b) aus dem Trageartikel (1) heraus weist und zur Ausbildung von Bergfalten (41, 42, 43), die konvex aus dem Trageartikel (1) heraus weisen und sich in der senkrechten Richtung (B) erstrecken, und von Talfalten (51, 52, 53), die konvex in den Trageartikel (1) hinein weisen und sich in der senkrechten Richtung (B) erstrecken, gefaltet ist, sodass die Bergfalten (41, 42, 43) und die Talfalten (51, 52, 53) einander in der Umfangsrichtung (C) abwechseln können und in der Umfangsrichtung (C) von dem gefalteten Zustand aus ausgedehnt werden können, wenn auf das Verbindungselement (6) in der Umfangsrichtung (C) ein Zug ausgeübt wird; und
die Außenoberfläche (13b) des Verbindungselements (6) an den Innenoberflächen (48, 49) des vorderen und des hinteren Hüftbereichs (2, 3) befestigt ist;
das Verbindungselement (6) aus einem Bahnmaterial (13) ausgebildet ist, das in einer Zick-Zack-Weise gefaltet ist, wobei ein Rand (20) innen in den Trageartikel (1) hinein weist, um mehrere Schichten (21-25) zu definieren, und eine erste Schicht (21) der Schichten (21-25) an der Innenoberfläche eines (9) der seitlichen Bereiche befestigt ist;
**dadurch gekennzeichnet, dass**
die mehreren Schichten (21-25) die erste Schicht (21) bis eine fünfte Schicht (25) umfassen;
die erste Schicht (21) mittels eines ersten Klebers (26) dauerhaft mit der Innenoberfläche des seitlichen Bereichs (9) verbunden ist und die fünfte Schicht (25) mittels eines zweiten Klebers (27) dauerhaft mit dem seitlichen Bereich (8) verbunden ist, und die vierte Schicht und die fünfte Schicht (25) mittels eines dritten Klebers (28) dauerhaft miteinander verbunden sind; und
die erste Schicht (21) bis dritte Schicht (23) des Bahnmaterials (13) in einem ersten Bereich (31) vorübergehend zusammengefügt sind und die erste Schicht (21) bis vierte Schicht (24) des Bahnmaterials (13) in einem zweiten Bereich (32) vorübergehend zusammengefügt sind.

2. Trageartikel gemäß Anspruch 1, wobei das Verbindungselement (6) mindestens zwei Bergfalten (41, 42, 43) aufweist.

3. Trageartikel gemäß Anspruch 1 oder 2, wobei das Verbindungselement (6) in der Umfangsrichtung (C) elastisch dehnbar ist.

4. Trageartikel (1) gemäß einem der Ansprüche 1 bis 3, wobei die Bergfalten (41, 42, 43) des Verbindungselements (6) in einem gefalteten Zustand weiter innen liegen als entsprechende Ränder der seitlichen Bereiche (8, 9) des entsprechenden vorderen und hinteren Hüftbereichs (2, 3), die sich in der senkrechten Richtung erstrecken.

5. Trageartikel gemäß einem der Ansprüche 1 bis 4, wobei zwei oder mehr Typen des Verbindungselements (6), die in dem in der Umfangsrichtung (C) gedehnten Zustand unterschiedliche Abmessungen haben, dazu verwendet werden, den vorderen und den hinteren Hüftbereich (2, 3) zu verbinden, um zwei oder mehr Typen des Trageartikels (1) zu erhalten, die unterschiedliche Umfangsabmessungen haben, und dadurch eine Gruppe zu erhalten, die die zwei oder mehr Typen des Trageartikels (1) enthält.

## Revendications

1. Article à porter de type culotte (1) ayant une direction circonférentielle (C), une direction avant-arrière (A) et une direction verticale (B) orthogonale à la direction avant-arrière (A), l'article à porter de type culotte (1) comprenant une région de taille avant (2) et une région de taille arrière (3) opposées entre elles dans la direction avant-arrière (A) et ayant respectivement des régions latérales (8, 9) opposées entre elles dans la direction circonférentielle (C) le long de laquelle la région de taille avant (2) et la région de taille arrière (3) sont raccordées ensemble, dans lequel :
la région latérale (8) de la région de taille avant (2) et la région latérale (9) de la région de taille arrière (3) sont raccordées entre elles par l'intermédiaire d'un élément de raccordement de type feuille (6) le long d'au moins l'un des deux côtés dans la direction circonférentielle (C) ;
l'élément de raccordement (6) a sa surface externe (13b) orientée vers l'extérieur de l'article à porter (1) et est plié afin de former des plis en hauteur (41, 42, 43) orientés de manière convexe vers l'extérieur de l'article à porter (1) et s'étendant dans la direction verticale (B) et des plis en creux (51, 52, 53) orientés de manière convexe vers l'intérieur de l'article à porter (1) et s'étendant dans la direction verticale (B) de sorte que les plis en hauteur (41, 42, 43) et les plis en creux (51, 52, 53) peuvent alterner dans la direction circonférentielle (C) et peuvent être expansés dans la direction circonférentielle (C) à partir de l'état plié lorsque l'élément de raccordement (6) est tiré dans la direction circonférentielle (C) ; et
la surface externe (13b) de l'élément de raccordement (6) est fixée sur la surface interne (48, 49) des régions de taille avant et arrière (2, 3) ;
l'élément de raccordement (6) est formé avec un matériau en feuille (13) qui est plié en zigzag avec un bord (20) orienté vers l'intérieur de l'article à porter (1) afin de définir une pluralité de couches (21-25), et une première couche (21) desdites couches (21-25) est fixée à la surface interne de l'une (9) des régions latérales ;
**caractérisé en ce que** :
ladite pluralité de couches (21-25) comprennent de la première couche (21) à une cinquième couche (25) ;
la première couche (21) est reliée de manière permanente à la surface interne de la région latérale (9) avec un premier adhésif (26) et la cinquième couche (25) est reliée de manière permanente à la région latérale (8) avec un deuxième adhésif (27), et la quatrième couche (24) et la cinquième couche (25) sont reliées de manière permanente entre elles avec un troisième adhésif (28) ; et
la première couche (21) à la troisième couche (23) du matériau en feuille (13) sont assemblées temporairement dans une première région (31) et la première couche (21) à la quatrième couche (24) du matériau en feuille (13) sont assemblées temporairement dans une seconde région (32).

2. Article à porter selon la revendication 1, dans lequel l'élément de raccordement (6) comprend au moins deux plis en hauteur (41, 42, 43).

3. Article à porter selon la revendication 1 ou 2, dans lequel l'élément de raccordement (6) est élastiquement extensible dans la direction circonférentielle (C).

4. Article à porter (1) selon l'une quelconque des revendications 1 à 3, dans lequel les plis en hauteur (41, 42, 43) de l'élément de raccordement (6) dans un état plié, sont à l'intérieur par rapport aux bords respectifs des régions latérales (8, 9) des régions de taille avant et arrière (2, 3) respectives s'étendant dans la direction verticale (B).

5. Article à porter selon l'une quelconque des revendications 1 à 4, dans lequel deux types ou plus de l'élément de raccordement (6) ayant des dimensions différentes à l'état expansé dans la direction circonférentielle (C) sont utilisés pour raccorder les régions de taille avant et arrière (2, 3) pour obtenir deux types ou plus d'article à porter (1) ayant différentes dimensions circonférentielles et obtenir ainsi un groupe comprenant les deux types ou plus de l'article à porter (1).
